Europäisches Patentamt

European Patent Office

Office européen des brevets

(19)

(11) Publication number: **0 505 627 A2**

# EUROPEAN PATENT APPLICATION

(12)

(21) Application number: **91202938.6**

(22) Date of filing: **12.11.91**

(51) Int. Cl.5: **G06F  15/42**, **A61B 5/00**

(30) Priority: **29.03.91 US 677798**

(43) Date of publication of application:
**30.09.92 Bulletin  92/40**

(84) Designated Contracting States:
**DE FR GB NL**

(71) Applicant: **ANALOGIC CORPORATION**
**8 Centennial Drive**
**Peabody, Massachusetts 02173(US)**

(72) Inventor: **Neumann, Leopold**
**9 Woodpark Circle**
**Lexington, Massachusetts 02173(US)**

(74) Representative: **Horton, Andrew Robert Grant**
**et al**
**BOWLES HORTON Felden House Dower**
**Mews High Street**
**Berkhamsted Hertfordshire HP4 2BL(GB)**

(54) Patient monitoring system.

(57) A patient monitoring system with integrated physiological data monitoring, patient visual image monitoring and two way audio communication over a communications network includes a first bedside station and a second remote central station. The first bedside station includes means for sensing physiological data from the patient; means for generating a video image of the patient and also includes an audio sensing and reproduction channel. The station includes means responsive to the means for sensing physiological data and the means for generating a video image and the means for sensing audio, for combining together and communicating the physiological data, video data and audio data and also for receiving and reproducing audio data. The second central station includes means for receiving and displaying together the combined video image and physiological data and for reproducing the audio and also means for sensing audio and communicating audio data to the bedside station. The system also may include consultive stations allowing remote patient examination, as described, and also may include a network based ambulatory-patient telemetry monitoring and location capability.

Fig. 1

## FIELD OF INVENTION

This invention relates to a patient monitoring system with integrated image data and physiological data communication and audio data communication, and more particularly to a patient monitoring system in which the remote bedside stations are interconnected through an interactive, integrated communication network with one or more remote central and consultative stations. The invention also includes a network based ambulatory-patient telemetry monitoring and location system.

## BACKGROUND OF INVENTION

Early patient monitoring was accomplished by electronic equipment maintained at the patient's bedside. Vital signs derived from physiological waveforms were monitored with the bedside equipment and alarms were generated if predetermined limits were exceeded by the vital signs. This bedside monitoring equipment became larger, more complex and expensive as each bedside unit undertook to monitor more physiological data and provide more sophisticated displays, e.g. color, more and better communications and more in-depth analysis of the data, such as calculation of vital signs and trends which required memory and processing capability. The provision of such units at each appropriate patient bedside introduces considerable additional expense to the hospital patient care costs.

With the introduction of bedside monitoring units, attempts were made to provide a measure of remote monitoring by transmitting analog waveforms of physiological data from the bedside unit to equipment at a central station such as a nurse's station. Subsequently remote monitoring efforts included analog waveforms plus digital representations for display. Both the bedside and remote monitoring activity acted to give alarms upon sensing an abnormal condition and to store data and analyze data to obtain vital signs and trends. But these systems are basically one-way systems reporting physiological data from the patient. There is no communication with the patient as a part of an interactive integrated system.

## SUMMARY OF INVENTION:

It is therefore an object of this invention to provide an improved interactive patient monitoring system.

It is a further object of this invention to provide such an improved patient monitoring system in which physiological data and video images are integrated in communication and display.

It is a further object of this invention to provide such an improved patient monitoring system which enables reduction of the equipment at the bedside station.

It is a further object of this invention to provide such an improved patient monitoring system which results in smaller, less complex and less expensive bedside units and lower overall system cost.

It is a further object of this invention to provide continuous routine visual surveillance and audio communication with a patient without continuously visiting the bedside for said purpose, thus enabling a nurse to provide better care for more patients.

It is a further object of this invention to provide such an improved patient monitoring system which enables consultive interchange between a remote consultant and a patient and/or patient attendant such as a nurse or physician present with the patient.

It is a further object of this invention to provide such an improved patient monitoring system which includes visual patient images and which transmits physiological data and video images together over the same network.

It is a further object of this invention to provide means to monitor via telemetry the physiological data of an ambulatory patient and to locate that patient using intelligent telemetry receiver/transmitter nodes located on the same network.

The invention results from the realization that a truly comprehensive patient monitoring system can be constructed by communicating integrated physiological data and video images together between a bedside monitor station and a remote monitor station, that the integrated images, audio, and data can be transmitted over the same communications network which can provide other services in the hospital and that a truly consultive, provisional examination capability can be effected using these features combined with an integrated audio channel to permit a physician or consultant to remotely preliminarily examine a patient.

This invention features a patient monitoring system with integrated physiological and video communications. There is a first bedside station and a second remote central station. The first bedside station includes means for sensing physiological data from the patient and means for generating a video image of the patient. There are means responsive to the means for sensing physiological data and the means for generating a video image for combining together and communicating the video image and physiological data over a network. The second remote central station includes means for receiving and displaying together the combined video image and physiological data.

In a preferred embodiment the stations include a two-way audio system for interactive audio communication with the patient or a patient attendant contemporary with transmission of the physiological data and video image. The means for combining and communicating may include a fiber optic communication system which may include a fiber optic communications network. The means for combining and communicating may also include a microprocesser controller for operating the fiber optic communication systems. The means for receiving and displaying may include a video display device and an image display control. The means for receiving and displaying may also include a numeric/text/graphics control, a waveform display control, and an anti-alias curve generating circuit. The means for receiving and displaying may also include an alarm device for indicating that a predetermined limit has been reached.

The first bedside station may include means for displaying together the combined image and physiological data, and that means for displaying together may include a display device, an image display control, a numeric/text/graphics control, a waveform display control, and an anti-alias circuit. More generally, the invention features a patient monitoring system with a first bedside station including means for sensing physiological data from the patient, and a second remote station including means for displaying the physiological data. The stations are interconnected by network means for communicating the physiological data between the stations.

The invention also features a patient monitoring system having a first bedside station including means for sensing physiological data from a patient; a second remote central station including means for displaying the physiological data; and a fiber optic network means for communicating the physiological data between stations.

The invention also features a patient monitoring system with integrated physiological and video communication. There is at least a first bedside station and a second remote central station. The first bedside station includes means for sensing physiological data from the patient, means for generating a video image of the patient, and means responsive to the means for sensing physiological data and the means for generating a video image for combining together and communicating the video image and physiological data. The second central station may include first means for receiving and displaying together the combined video image and physiological data from each of the first stations. There may be a third consultive station responsive to at least the first patient station. The consultive station includes second means for receiving and displaying together the combined video image and physiological data from each of the first stations. There is an audio system for interactive audio communication between the stations contemporaneously with transmission of the physiological data and video image.

The invention also features a patient monitoring system with integrated physiological and video communication comprising at least a first bedside station and a second consultive station. The first bedside station includes means for sensing physiological data from the patient, means for generating a video image of the patient, and means responsive to the means for sensing physiological data and the means for generating a video image for combining together and communicating the video image and physiological data. The second consultive station is responsive to at least the first patient station and includes means for receiving and displaying together the combined video image and physiological data from each of the first stations. In a preferred embodiment there is further included an audio system for interactive audio communication between the stations contemporaneously for transmission of the physiological data and video image.

The invention also features a patient monitoring system with integrated physiological and video communication including at least bedside station, a central station and a consultive station. There is a local service station including an archival storage and retrieval medium and means responsive to one of the stations for retrieving from the archival medium historical patient data and transmission of it to and display at the other station. In a preferred embodiment the means for receiving and transmitting may include a fiber optic communications system.

The invention also features an ambulatory patient monitoring system. There is a communication system including a transceiver unit associated with each patient and including means for sensing physiological data from the patient. There is also a plurality of monitoring and location nodes including means for broadcasting through each node an identification of a patient to be monitored. There are means for interrogating the patient's transceiver unit through at least one of the nodes for acquiring the sensed physiological data. A central station is provided for receiving and displaying the physiological data from the communication system and there is a communication network for interconnecting the central station and the communication system. In a preferred embodiment the communication system may include means for re-actuating the means for broadcasting when the patient is out of range of the current node. The communication network may be a fiber optic communication network and the communication system may include an RF communication system.

## DISCLOSURE OF PREFERRED EMBODIMENT

Other objects, features and advantages will occur to those skilled in the art from the following description of a preferred embodiment and the accompanying drawings, in which:

Fig. 1 is a block diagram of a patient monitoring system according to this invention;

Fig. 2 is a more detailed block diagram of a bedside station and communications interface according to this invention;

Fig. 3 is a more detailed block diagram of the remote or central station of Fig. 1;

Fig. 4 is a more detailed block diagram of the consultive station of Fig. 1;

Fig. 5 is a more detailed block diagram of the consultive station of Fig. 4;

Fig. 6 is a more detailed block diagram of the central station of Fig. 3;

Fig. 7 is a more detailed block diagram of the bedside station of Fig. 2;

Fig. 8 is a simplified schematic block diagram of a local service station according to this invention;

Fig. 9 is an illustration of an image display typically appearing on a monitor at the central station;

Fig. 10 is an illustration of an image which typically can appear at the central station or a consultive station;

Fig. 11 is a schematic diagram of a comprehensive fiber optic network including a plurality of central stations, bedside stations, consulting stations, a local service station, and a patient telemetry unit according to this invention;

Fig. 12 is a simplified block diagram of the cellular node and a patient telemetry unit for an ambulatory patient station according to this invention; and

Fig. 13 is a flow chart illustrating the operation of an ambulatory patient telemetry unit.

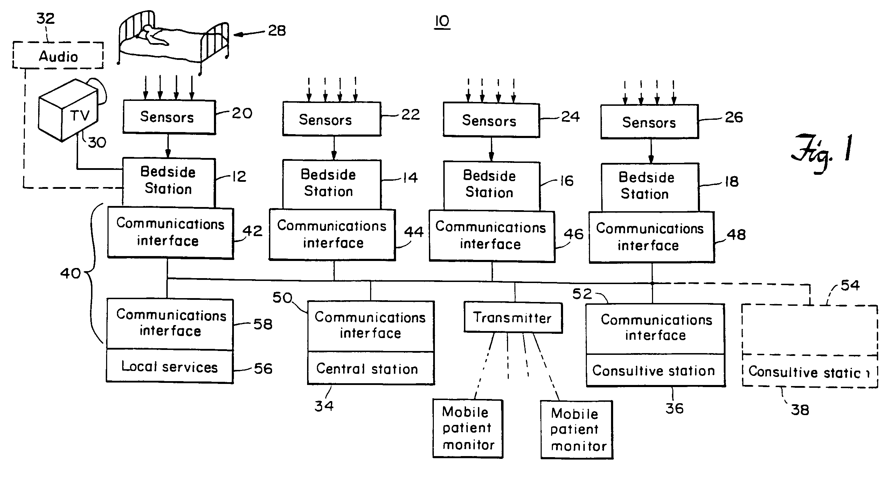

There is shown in Fig. 1 a patient monitoring system 10 according to this invention including a plurality of bedside stations 12, 14, 16 and 18, which receive physiological data via sensors 20, 22, 24 and 26 from a patient 28 (depicted only with respect to sensors 20 and bedside station 12). Each bedside station 12, 14, 16, 18 may include a video camera 30 as shown associated with bedside station 12, so that the patient can be viewed at any time and especially contemporaneously with the taking of physiological data. Each bedside station may also include an audio unit 32 shown associated with beside station 12 to permit two-way audio communication with patient 28. Bedside stations 12, 14, 16 and 18 communicate with at least one remote central (nurse's) station 34 and may communicate with one or more consultive stations 36, 38 over communications network 40. Each bedside station includes as a part of the network 40 a communications interface 42, 44, 46 and 48. Communications interfaces 50 52, and 54 are also provided for remote central (nurse's) station 34 and consultive stations 36 and 38, respectively. Local services such as X-ray images, patient records, and historical data may be provided from a local services system 56, which also includes a communication interface 58 as a part of communications network 40.

In operation, physiological patient 28 is constantly monitored through sensors 20. This data, in raw form and as processed to provide vital signs and trends, may be viewed at bedside station 12. In addition, that information may be communicated to remote central station 34 over network 40 and it may be communicated integrally with a video image of the patient derived from video system 30 and/or audio communications with the patient obtained through audio system 32. This remote central station may be a nurse's station or some other form of central station where doctors and nurses may wish to make a remote preliminary examination of the patient.

Simultaneously with the image of the patient on a screen the remote central station 34 may receive physiological data in numerical form and curves and graphs indicating vital signs and trends. In addition, X-ray images and historical data such as charts and records may be provided from the local services system 56. All of the information communicated to remote central station 34 may also be communicated to one or more consultive stations 36 and 38 so that a physician, specialist or other expert may be able to view and preliminarily or provisionally examine the patient through the physiological data, the patient's history, the vital signs and trends integrated with video and audio communication with the patient or a bedside attendant who is examining and questioning the patient.

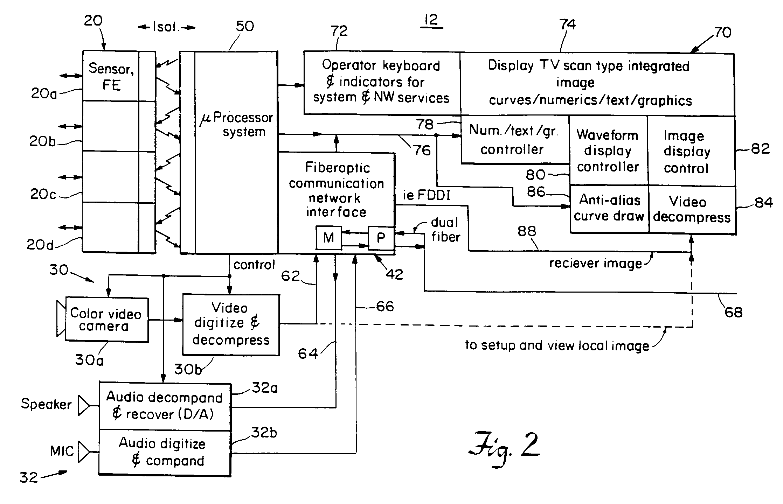

Bedside station 12, Fig. 2, uses a number of sensors 20a, 20b, 20c and 20d for sensing the physiological data in analog form and submitting it to microprocessor system 50. The outputs from sensor 20 may be immediately transformed to digital signals or that may be done when they arrive at microprocessor system 50. Video unit 30 may include a color video camera 30a and a video digitize and compress circuit 30b. Audio system 32 may include an audio decompand and recover circuit 32a and an audio digitize and compand circuit 32b. The outputs from video video unit 30 and audio unit 32 are provided on lines 62, 64 and 66 to communication interface 42, which may be a fiber optic communication network interface according to the FDDI standard. The video, audio and physiological data are combined and integrally transmitted by interface 42 over line 68 to remote central station 34. Optionally, bedside station 12 may include an interactive display unit such as plug-in display unit 70, which includes an operator keyboard 72 and an integrated patient display 74 which can provide images, numerics, text, curves and graphics all on the same screen and contemporaneously. Physiological data in its original form and converted to vital signs and trends by microprocessor 60, may be provided over line 76 to plug-in display unit 70, where a numeric/text/graphic controller 78, a waveform display controller 80, image display control 82, and video decompress circuit 84 process the information and display it on the screen of integrated patient display 74. Anti-alias curve drawing circuit 86 is provided to enhance the depiction of curves on display 74. The video signal is provided to video decompress circuit 84 via line 88 from fiber optic communications network interface 42.

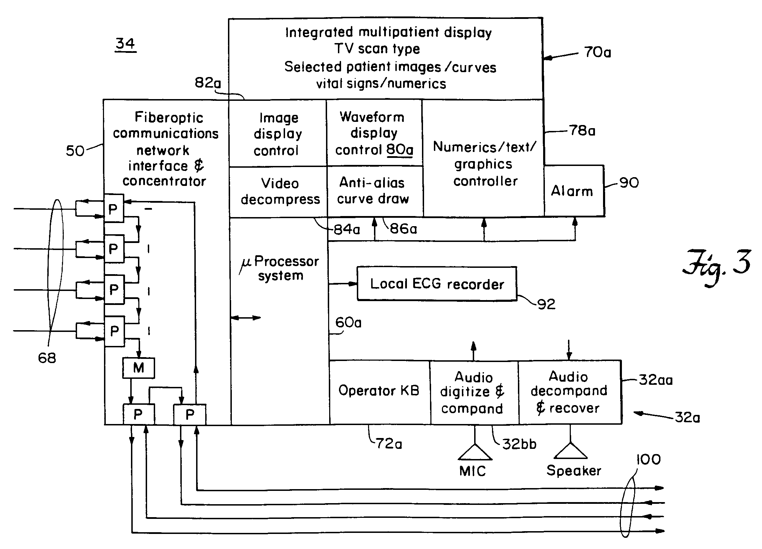

Central station 34, Fig. 3, such as a nurse's station or central station in a hospital, includes fiber optic communication network interface and concentrator 50. Station 34 also includes an integrated multi-patient display 70a similar to that shown in bedside station 12 except that it can simultaneously display the input from a number of different bedside stations. It also displays patient images, curves, vital signs and numerics. Station 34 also includes image display control 82a, waveform display controller 80a, numeric/text/graphics controller 78a, video decompress circuit 84a, and anti-alias curve drawing circuit 86a. Alarm 90 is provided to give audio/visual alarms if any physiological data or vital signs or trends derived therefrom exceed predetermined limits. Station 34 may also include a local ECG recorder 92 so that a patient's ECG and other data may be printed out locally at the central station 34. Station 34 also includes an operator keyboard 72a and audio system 32 including an audio-digitized and compand circuit 32bb and an audio decompand and recover circuit 32aa. Microprocessor system 60a functions as did system 60 in Fig. 2 to control the entire station.

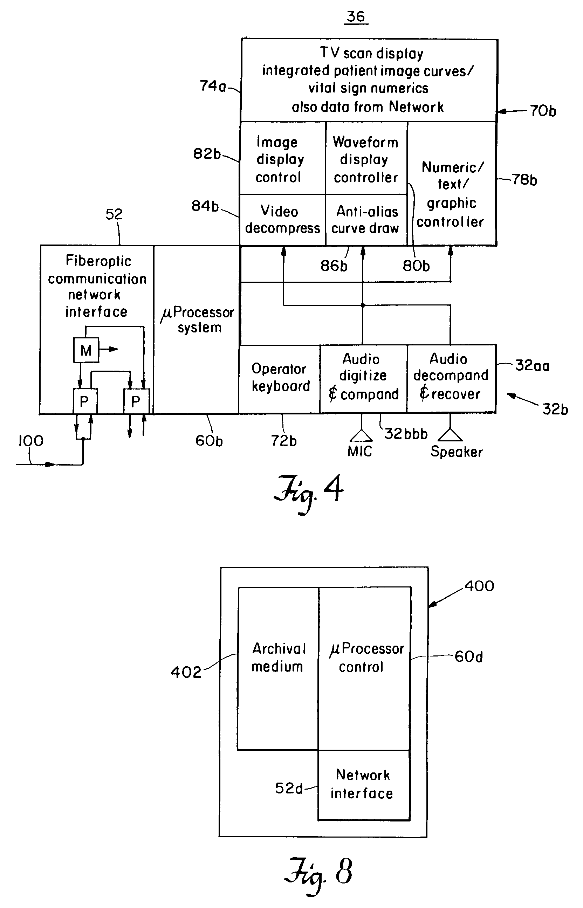

Central station 34 may communicate over network 100 with one or more consultant stations 36, Fig. 4. Consultant station 36 includes an interactive unit 70b including a display 74a, an image display control 82b, waveform display controller 80b, numeric/text/graphic controller 78b, video decompress circuit 84b, and anti-alias curve drawing circuit 86b. There is also an operator keyboard 72b. There is an audio system 32b including an audio-digitizing compand circuit 32bbb and an audio decompand and recover circuit 32aaa. Consultive station 36 communicates over line 100 with central station 34 through fiber optic communication network interface 52 and the entire station is operated and controlled by microprocessor system 60b.

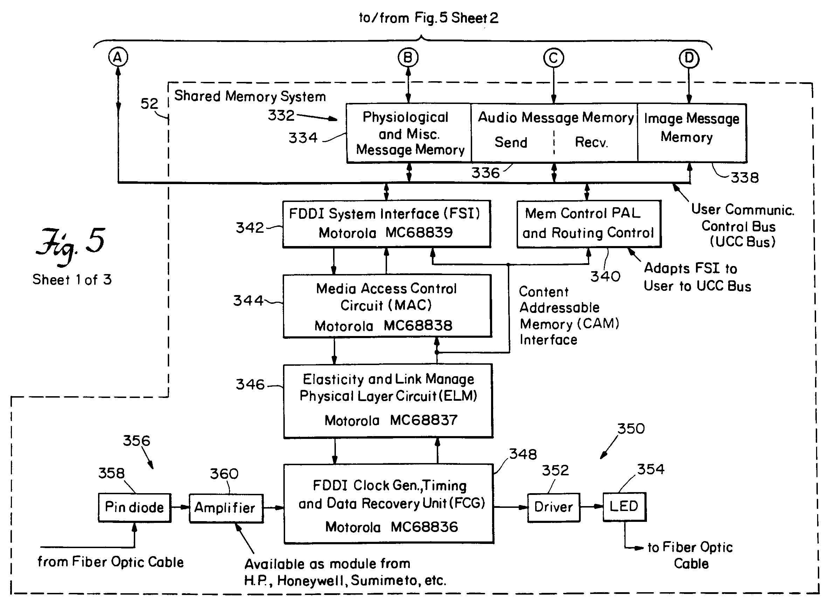

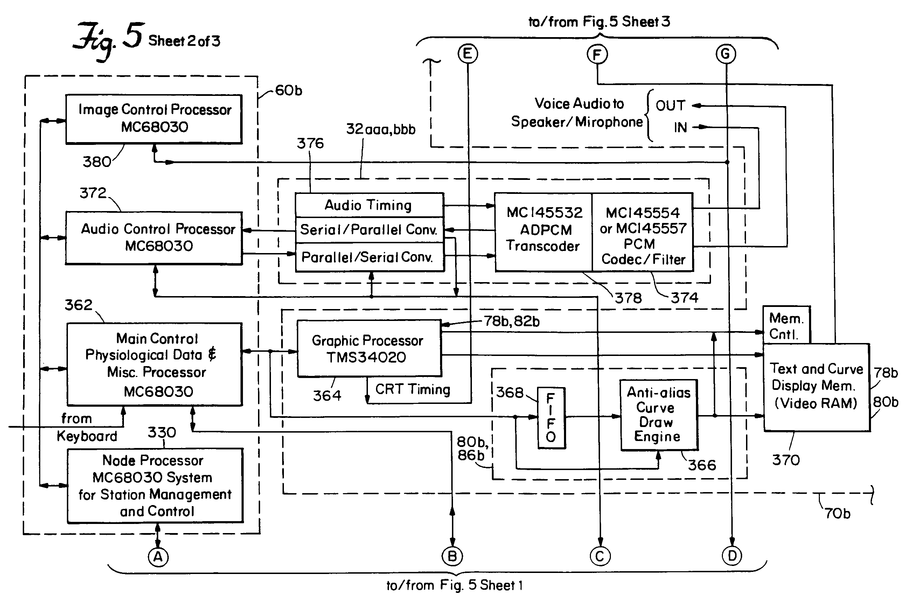

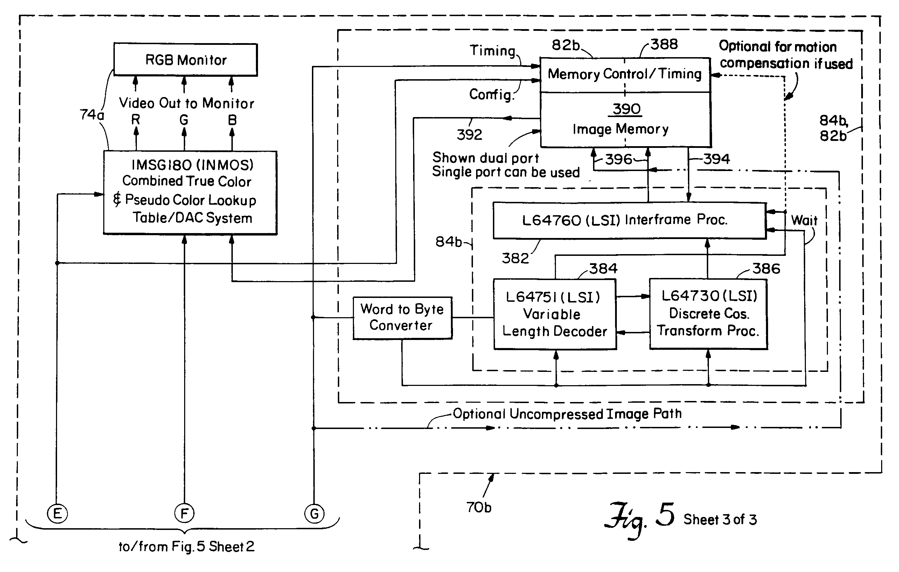

Consultive station 36 is shown in more detail in Fig. 5, where fiber optic network interface 52 is implemented in accordance with FDDI standards and based on a Motorola chip set MC68836-MC68839. The chip set is supported by node host processor system 330 which forms a part of microprocessor 60b. Node processor system 330 performs the station management in accordance with the FDDI SMD specification. Node processor system 330, the MC68030, is supported by ROM, RAM, interrupt controllers and interfaces in a standard manner. The chip set communicates with a shared memory system 332 which consists of a physiological and miscellaneous message memory 334, audio message memory 336, and image message memory 338, via direct memory access implemented by the chip set to obtain and to output control information and obtain and output message data in accordance with the chip set operation. Message header blocks are examined and used to route messages having a different destination address or subaddress to one of two, three or more separate shared memories for convenience in later processing. Also on transmit, messages may originate from one of two, three or more of the shared memories. Receive routing may be done using the message destination address. The implementation shown uses some external augmentation of the chip set content-addressable memory, such as memory control PAL 340, in conjunction with the node processor 330 to accomplish the routing. This may be simplified by adapting a subaddress format convention so that only a few bits of the address determine the routing. The remainder of network interface 52 is similar to the corresponding apparatus in the other systems. There is an FDDI system interface circuit 342, a media access control circuit 344, and an elasticity and link manage physical layer circuit 346 which responds to the FDDI clock generator timing and data recovery unit 348. Unit 348 interconnects with the transmitter 350 including driver 352 and LED 354, and also with the receiver 356 which includes pin diode 358 and amplifier 360.

The main control in the physiological data system using microprocessor 362 has its own set of standard resources. Processor 362 communicates with the physiological and miscellaneous message memory 334 to send and receive messages. Processor 362 services and interprets the user keyboard and controls an information menu display and a curve display working in conjunction with a slave graphics processor 364 which forms a part of numerical/text/graphics control 78b and image display control 82b. There is an anti-alias curve drawing engine 366 which in conjunction with first-in first-out storage circuit 368 forms a part of both waveform display control 80b and anti-alias curve drawing circuit 86b, which write to the text and curve display memory 370 that forms a portion of both numeric/text/graphic control 78b and waveform display control 80b.

An audio system using audio control processor 372 has its own set of standard resources. Processor 372 controls the transfer of information to a PCM codec filter 374 via parallel to serial converter interface 376 as the codec selected takes and outputs serial data. An ADPCM transcoder 378 may be used in conjunction with the interface 376 to reduce the amount of audio information to be transmitted and received, for example from 64 KB/sec to 32 KB/sec. The video image display uses control microprocessor 380 having its own set of standard resources. Processor 380 controls an LSI logic image decompression chip set 84b which includes an interframe processor 382, variable length decoder 384, and a discrete cosine transform processor 386. Microprocessor 380 also controls supporting memory system 82b, 84b, which includes memory control timing 388 and image memory 390. The LSI logic image decompression set 84b acts on a coded data stream which has been received as a series of blocks in the image message memory 390 to produce and/or update an image in the image memory 390. For simplicity the image memory 390 may be duplicated to become two two-port memories rather than a three-port memory. One port 392 is display output, one port 394 is output to support interframe coding, and one port 396 (duplicated) is input from decompression. The display output port memory control/timing may be configured to support a number of image display formats to allow the image to be placed appropriately on the display CRT and be magnified or shrunk as selected. The input port 396 may be configured optionally to support a motion compensation function in the compression; however, this may not be required for the type of images expected to be handled in the system. All the processors in the system communicate with one another. The communication may be implemented in one of a number of ways. Typically the communication is via messages placed in a shared memory 332 augmented by interrupt. Communication between the main control and physiological data processor 362 and the audio processor 372 and image processor 380 will pass through the node processor 330. The role of the microprocessor system 60b in the audio and the image system is minimal. In most systems they can be eliminated and physically replaced by one of the other processors. They function to initialize the subsystems and to control or initialize the transfer of block message data to and from the output/input systems.

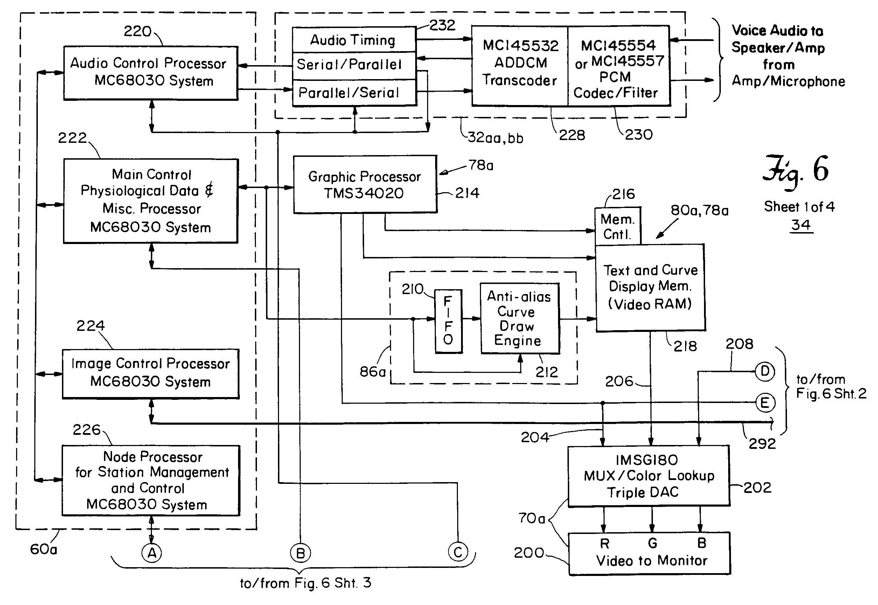

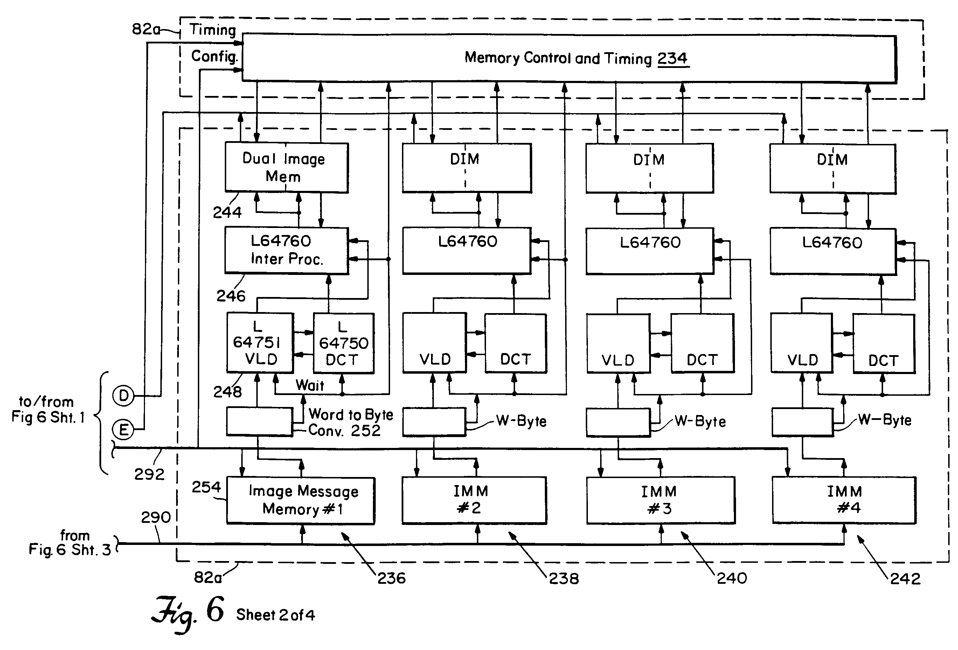

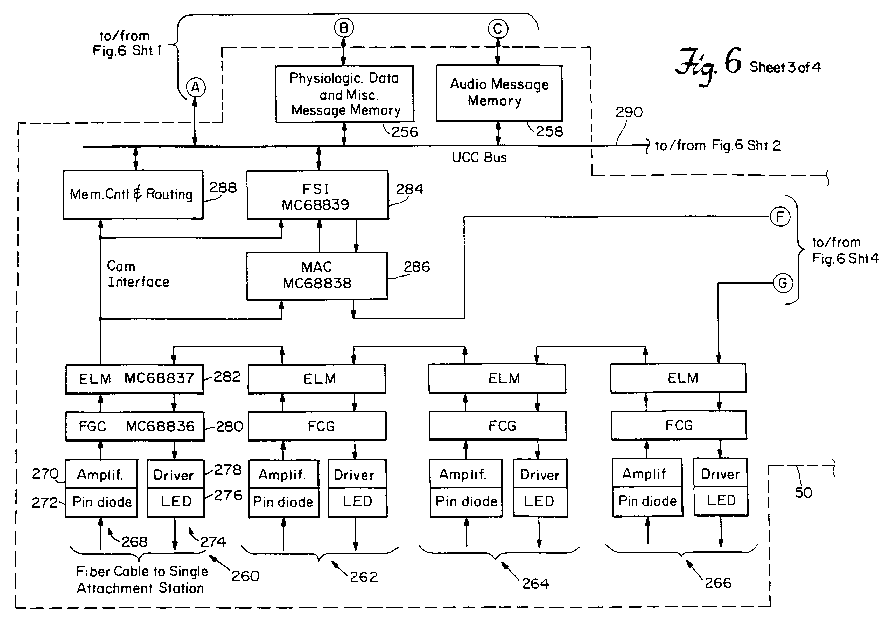

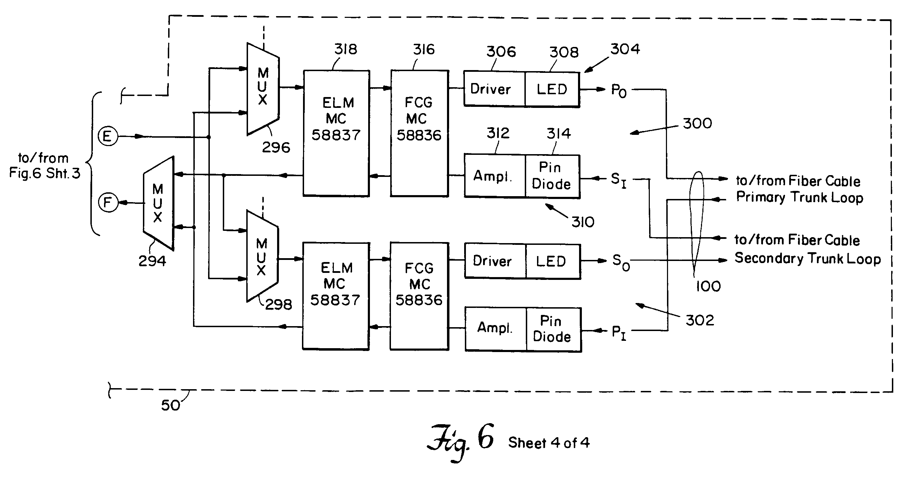

The nurse's or central station 34 is shown in greater detail in Fig. 6, where the integrated multi-patient display 78 includes a red-green-blue monitor 200 driven by a multiplexer color lookup triple digital-to-analog converter 202. In addition to the timing input 204, digital-to-analog converter 202 receives three data inputs: the text and graphic data on line 206 and the image data on line 208. Anti-alias curve drawing circuit 86a includes a first-in first-out storage 210 and anti-alias curve drawing engine 212, which provides an apparent better resolution than can the rest of the display by writing two appropriate intensities in adjacent pixels. Numeric/text/graphics controller 78 is implemented in graphic processor 214, memory controller 216, and text and curve display memory 218, the latter of which also functions as the waveform display controller 80a. Microprocessor 60a actually includes four microprocessors: audio/video control processor 220; main control physiological data and miscellaneous processor 222; image control processor 224; and node processor 226. Audio digitize and compand 32bb and audio decompand and recover 32aa are constructed using transcoder 228 and a pulse code modulator decoder 230 in conjunction with an audio timing circuit 232 that provides serial-parallel output.

Image display control 82a includes memory control and timing circuit 234, and video decompress circuit 84a includes four decompression channels 236, 238, 240, and 242. As exemplified by channel 236, each channel includes a dual-image memory 244 wherein one bank of memory supports the decompression algorithm and the other memory supports the video refresh. A single chip set includes interframe processor 246, variable length decoder 248, and discrete cosine transform 250. The remainder of channel 236 includes word-to-byte converter 252 and image message memory 254. Word-to-byte converter 252 (and 182 in Fig. 5) functions to convert between the long words used in the communication system interface and the byte data used in the video compression/decompression ship set.

Image message memory 254 in channel 236 and corresponding memories in channels 238, 240 and 242, along with the physiological data and miscellaneous message 256 and audio message memory 258 function as buffer memories in a fiber optic communications interface concentrator 50. There are one or more ports 260, 262, 264, 266, for interconnection with a bedside monitor or station 12. Each port, as represented at port 260, includes a receiver 268 having an amplifier 270 and pin diode 272, and a transmitter 274 which includes an LED 276 and a driver circuit 278. Also associated with each port is an FDDI clock generator 280 and an elasticity and link management physical layer circuit 282 which is a portion of a Motorola FDDI chip set. Other elements of the chip set which are shared include the FDDI system interface 284 and media access control circuit 286. Memory control and routing circuit 288 supervises activities on UCC bus 290. Bus 290 and bus 292 serve dual port memories 256, 258 and 254. Multiplexers 294, 296, 298 function to interconnect the bedside stations at ports 260-266 with the main fiber optic network interconnected through dual loops 100. Multiplexers 296 and 298 actually function to connect the bedside station to one of the two loops 300, 302 of the dual-loop network 100. Each dual-loop as represented by dual loop 300 includes a transmitter 304 including a driver circuit 306 and LED 308, and a receiver 310 including amplifier 312 and a pin diode 314. Loop 300 also includes an FDDI clock generator 316 and elasticity and link-managment physical layer circuit 318 as previously discussed.

The central station shown is a dual attachment station configured as an FDDI concentrator for four (4) single attachment stations, which are bedside stations, or other single attachment central stations, or concentrators. The concentrator circuitry includes multiplexers allowing the station to connect to either the primary or the secondary trunk of an FDDI network and also allows disconnecting the station from the network (via bypass), while maintaining communication with the single connection station monitoring loop. The Motorola MC68837's have provision for bypassing unconnected stations at the concentrator.

The illustrated central station supports separate decompression and display of four (4) patient images (this need not be equal to the concentrator capability) which may be selected from the various bedsides and displayed on a high-resolution display, along with curves, numerical data, menus, etc. The implementation shown is a maximalist approach, duplicating memories and decompression circuitry four (4) times. The circuitry may be simplified by sharing hardware, especially at lower transmitted frame rates.

The system, as shown, supports a two-way audio link but may, if desired, be augmented by the ability to simultaneously receive two or more audio sources and mix them.

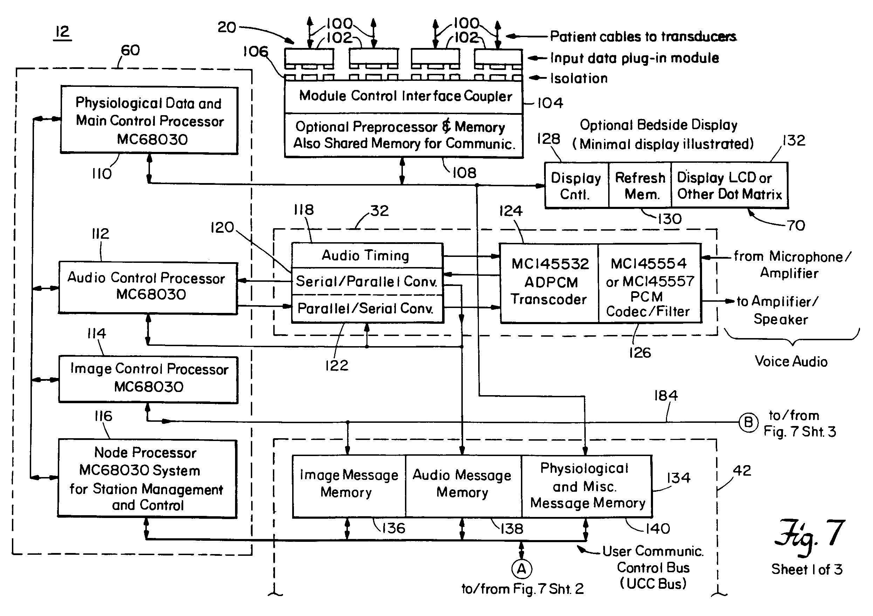

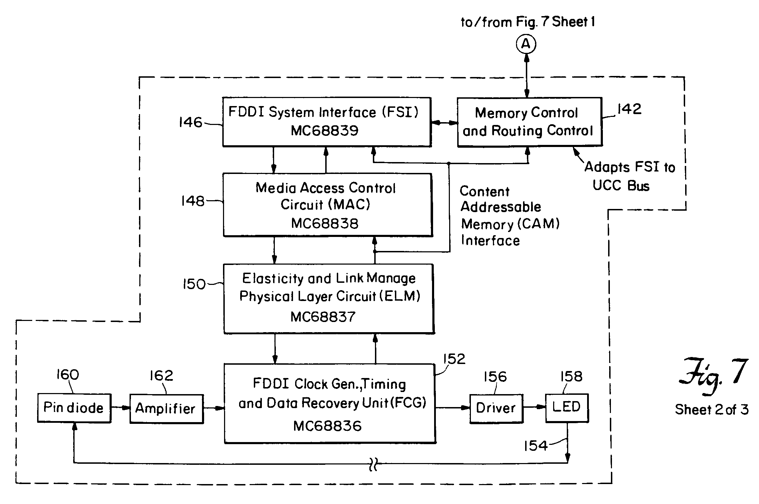

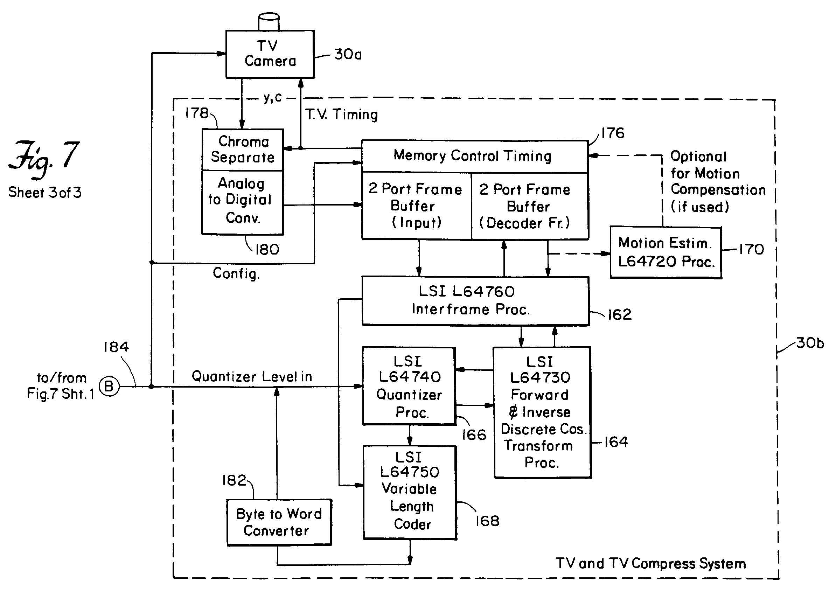

Bedside station 12, along with its sensors 20, communication interface 42, television 30 and audio 32, are shown in greater detail in Fig. 7, where sensor 20 includes a plurality of patient cables 100 which connect to transducers at the patient at one end and at the other end connect to input data plug-in modules 102. Each cable 100 is connected to a different transducer such as for sensing blood pressure, temperature, ECG and respiratory waveform. The plug in modules 102 interconnect with the module control interface coupler 104 via isolation units 106 which may be electro-optic isolators, transformers, or other devices, or a combination of them. Module control interface coupler 104 connects directly to an optional preprocessor and memory 108 which communicates with microprocessor 60. Such a system is disclosed in greater detail in U.S. Patent No. 4,409,669, issued October 11, 1983, to the same inventor and is incorporated herein by reference.

Microprocessor 60 actually includes four microprocessors -- a physiological data and main control processor 110, an audio control processor 112, image control processor 114, and node processor system 116, each implemented by an MC 68030 chip. Audio portion 32 includes an audio timing circuit 118, serial to parallel converter 120, and a parallel to serial converter 122, which interface with an AD3CM transcoder 124 implemented by an MC 145532, and a pulse code modulated filter and codec 126 implemented by an MC 143554 or 145557. Display unit 70 is shown in more simplified form in Fig. 5 as including display control 128, a refresh memory 130, and an LCD display 132, which is typically a single integrated circuit which can be obtained as a single product.

Fiber optic interface 42 includes a number of dual port memories 134 including an image message memory 136, audio message memory 138 and physiological and miscellaneous message memory 140. Memory control and routing control 142 determine which memory is to be communicated with and accommodates the particular user communication control bus 144 that is employed which would be compatible with the MC 68030 chip that implements the four microprocessors. The electro-optic interface includes four chips: an FDDI system interface 146 implemented by an MC 68839 chip, a media access control circuit 148 implemented by an MC 68838 chip, an elasticity and link manage physical layer circuit 150 implemented by an MC 68837 chip, and an FDDI clock generator timing and data recovery unit 152 implemented by an MC 68836 chip, which together form an interface chip set.

The fiber optic cable 154 is connected with unit 152 using conventional interconnection hardware such as driver 156 and LED 158, and a pin diode 160 and amplifier 162.

Video digitize and compress circuit 30b includes an LSI processing chip set 160 which includes interframe processor 162 implemented by an L64760, a forward and inverse discrete cosine transform processor 164 implemented by LSI 64730, the quantizer processor 166 implemented by an L64740, a variable length coder 168 implemented by an L64750, and a motion estimator processor 170 implemented by an L64720. Chip set 160 operates in accordance with protocol CCITT H.261 "Standard", which compresses the video data for lower bandwidth transmission utilizing two-port frame buffer 172 and two-port frame buffer 174. Memory control and timing 176 is compatible with the chip set 160 requirement and the video rate. Input from TV camera 30a to frame buffer 172 is through a chroma separation circuit 178 and an analog to digital converter 180. Byte to word converter 182 converts the output from chip set 160 from byte to word format compatible with memory 134 on line 184. Line 184 also provides a quantizer level to quantizer processor 166 in accordance with the algorithm informing it of the utilization of the bandwidth capacity so that chip 160 can operate at a higher or lower data rate in order to fully utilize memory 134.

The bedside monitor includes a modular, isolated, physiological-data front-end which may be as described in U.S. Patent No. 4,409,669.

Bedside station 12 generally includes a patient image acquisition and an image compression system. A television camera, such as the Panasonic WV-CL700 intended for CCTV usage, may be used for image acquisition or an alternative camera may be used.

The camera may output an analog signal to the system (i.e., NTSC composite, S video Y,C signals (as the WC-CL700 or RGB), in which case adapter circuitry is required to produce a digital version of the luminance and the two chroma color-difference signals needed for the compression engine. Alternately, a camera having direct digital outputs may be used.

S video cameras such as the WV-CL700 utilize digital processing internally so they could easily be modified to have a direct digital output. A compression engine using an LSI Logic chip set, which is compatible with the CCITT-teleconferencing standard "H.261", is illustrated. The image compression operation considerably reduces the amount of data that must be transmitted to obtain a high-quality moving image.

The system illustrated also includes a minimal bedside display intended to display derived patient numerical parameters (i.e., heart rate, blood pressure, temperature, etc.) and low-resolution waveforms. A dot-matrix, liquid crystal display system is illustrated., but a high-resolution display or even a color display could be used.

The system also includes a two-way "ADPCM", compressed audio system, and an FDDI interface. The bedside station has been configured as a "single attachment" FDDI station which attaches to the network via a "concentrator". Also, note that the consultive station was also configured as a "single attachment" FDDI station, which attaches to the network via a concentrator.

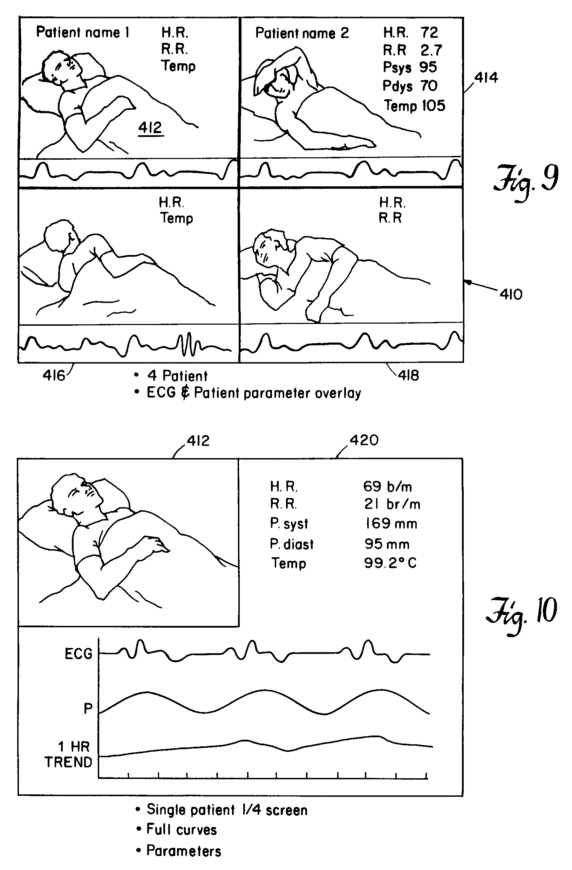

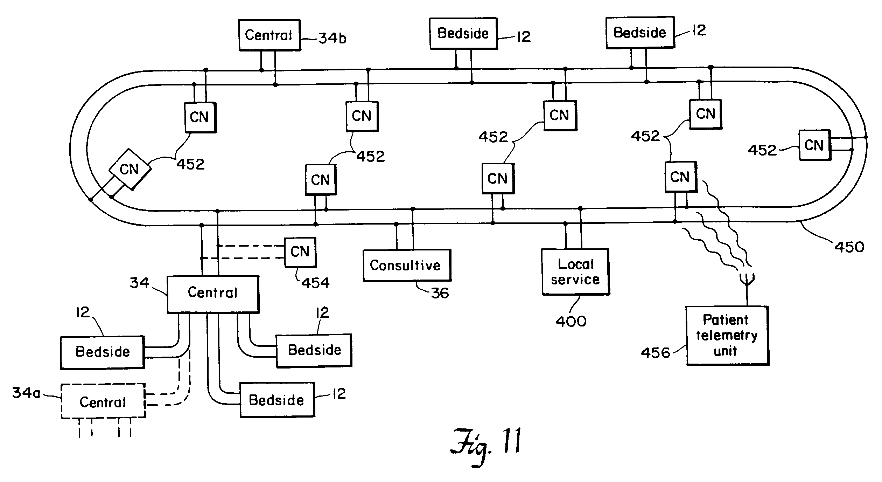

The system can be provided with additional services by a local service station 400, Fig. 8, which for example can serve to provide the archival or historical records of a patient. A local service station would be constructed in the same way as the other stations with a network interface 52d and microprocessor control 60d and all of the other necessary controls as described in Figs. 1-7, all associated with the archival storage medium 402. Local station 400 is accessible to any station on the network, including the central station, the consultive station, and even the bedside stations. A variety of displays may be provided on the monitors of the various stations. The central station may have a display 410, Fig. 9, which includes four quarter images 412, 414, 416, and 418, of four different patients monitored by four different bedside stations. Each image includes an image of the patient as well as the patient name, his most important curves and data. If the nurse or doctor at the central station wished for more information about a specific patient, then the display 420, Fig. 10, might be used, where the quarter image 412 of the patient is retained but additional information in the curve area and the alphanumeric data is provided. At a consultive station where a full and larger view of the patient himself may be desirable during a remote examination, the full screen can be occupied with the image with a small amount of alphanumeric data and one or two curves. The consultive station could as well receive an image such as 420, Fig. 10, when desired. Other images available include simply curve and alphanumeric data for only one or a number of patients, or curve and alphanumeric data on a single patient. Various combinations of these may be used by different stations at different times as appropriate. Typically the central station is the only station that is provided with multiple images such as shown in Fig. 9. In alarm conditions, such as where heart rate goes outside of limits, the system may automatically sound audio alarms, flash visual alarms, and may also automatically switch the central station screen so that it contains a full screen concerning only that patient causing the alarm condition.

Central station 34 may be interconnected with one or more consultive stations 36 and local service stations 400 over a dual-loop FDDI network 450, Fig. 11. Central station 34 typically has a plurality of bedside stations 12 interconnected through a single loop and may also have one or more additional central stations 34a in addition to or in place of the bedside stations 12. Network 450 may have additional central stations 34b and may also interconnect directly with bedside stations 12 which have been provided with dual-loop capacity. Also included on network 450 may be a plurality of cellular communication nodes 452 which may be connected directly to network 450 or may be connected to other devices which in turn connect to network 450. For example, cellular node 454 interconnects through central station 34 to network 450.

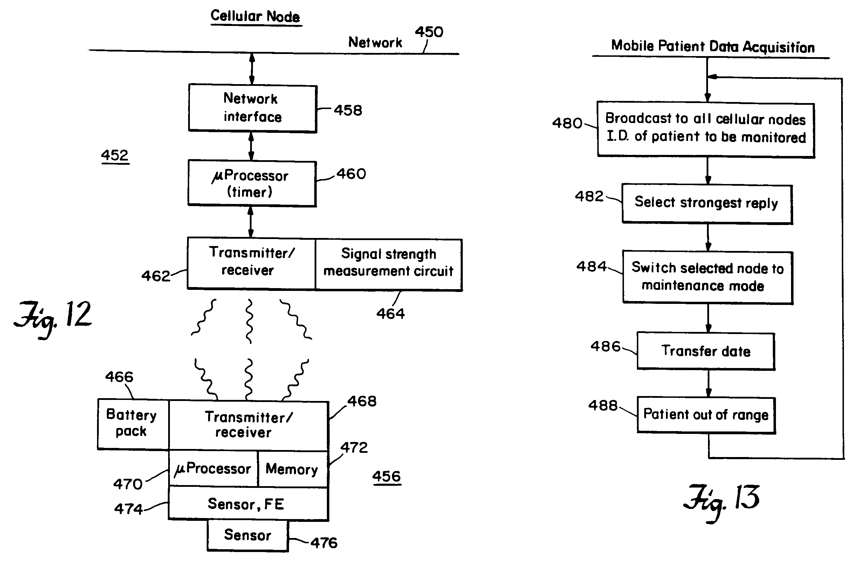

By providing a patient with a patient telemetry unit 456, the cellular nodes 452 can be used to locate a patient as present in at least the area of the controlling node and the patient within that area can have his telemetry unit 456 interrogated to transfer vital body signs to the various stations. Each node typically includes network interface 458, Fig. 12, for interfacing with network 450, a microprocessor and timer 460, transmitter and receiver 462, similar to those used in the other stations, and a signal strength measurement circuit 464 for determining whether or not the patient telemetry unit 456 is within the field served by this particular cellular node. Patient telemetry unit 456 is operated by a battery pack 466 and includes a transmitter-receiver 468, microprocessor 470, memory 472, and a sensor front end 474, plus a sensor 476.

Mobile patient data acquisition is accomplished by first broadcasting to all cellular nodes the ID of a patient to be monitored in step 480, Fig. 13. The station then selects the strongest reply, step 482, and switches the selected node to a maintenance mode in step 484 to monitor that patient. On demand or cyclically the data can be transferred from that patient in step 486 via the patient telemetry unit 456. The data transfer occurs through the patient telemetry unit 456 to the supervisory one of nodes 452, and then through the network 450 to whichever station is calling for it. If the patient is out of range, the signal is sent back and step 480 is inaugurated again.

Although specific features of the invention are shown in some drawings and not others, this is for convenience only as each feature may be combined with any or all of the other features in accordance with the invention.

Other embodiments will occur to those skilled in the art and are within the following claims:

## Claims

1. A patient monitoring system with integrated physiological and video communication comprising:

a first bedside station and a second, remote station;

said first bedside station including:

means for sensing physiological data from the patient;

means for generating a video image of the patient; and

means, responsive to said means for sensing physiological data and said means for generating a video image, for combining together and communicating the video image and physiological data;

said second, central station including means for receiving and displaying together said combined video image and physiological data.

2. The patient monitoring system of claim 1 in which said stations further include a two-way audio system for interactive audio communication with the patient or a patient attendant contemporary with the transmission of the physiological data and video image.

3. The patient monitoring system of claim 1 in which said means for combining and communicating includes a fiber optic communications system.

4. The patient monitoring system of claim 3 in which aid fiber optic communications system includes a fiber optic communications network.

5. The patient monitoring system of claim 3 in which said means for combining and communicating includes a microprocessor controller for said fiber optic communications systems.

6. The patient monitoring system of claim 1 in which said means for receiving and displaying includes a display device.

7. The patient monitoring system of claim 6 in which said means for receiving and displaying includes a video image display control.

8. The patient monitoring system of claim 7 in which said means for receiving and displaying includes a numerics/text/graphics control.

9. The patient monitoring system of claim 8 in which said means for receiving and displaying includes a waveform display control.

10. The patient monitoring system of claim 9 in which said means for receiving and displaying includes an anti-alias curve generation circuit.

11. The patient monitoring system of claim 1 in which said means for receiving and displaying includes an alarm device for indicating that a predetermined limit has been reached.

12. The patient monitoring system of claim 1 in which said first station includes means for displaying together said combined image and physiological data.

13. The patient monitoring system of claim 12 in which said means for displaying together includes a display device.

14. The patient monitoring system of claim 13 in which said means for displaying together includes an image display control.

15. The patient monitoring system of claim 14 in which said means for displaying together includes a numeric/text/graphics control.

16. The patient monitoring system of claim 15 in which said means for displaying together includes a waveform display control.

17. The patient monitoring system of claim 16 in which said means for displaying together includes an anti-alias circuit.

18. A patient monitoring system comprising:
    a first bedside station including means for sensing physiological data from the patient;
    a second, remote central station including means for displaying said physiological data; and
    fiber optic network means for communicating said physiological data between said stations.

19. A patient monitoring system with integrated physiological and video communication comprising:
    at least a first bedside station and a second, remote central station;
    said first bedside station including:
    means for sensing physiological data from the patient;
    means for generating a video image of the patient; and
    means, responsive to said means for sensing physiological data and said means for generating a video image, for combining together and communicating the video image and physiological data;
    said second, central station including first means for receiving and displaying together said combined video image and physiological data from each of said first stations;
    a third, consultive station, responsive to at least said first bedside station and including second means for receiving and displaying together said combined video image and physiological data from each of said first stations; and
    an audio system for interactive audio communication between said stations contemporaneously with transmission of the physiological data and video image.

20. A patient monitoring system with integrated physiological and video communication comprising:
    at least a first bedside station and a second, consultive station;
    said first bedside station including:
    means for sensing physiological data from the patient;
    means for generating a video image of the patient; and
    means, responsive to said means for sensing physiological data and said means for generating a video image, for combining together and communicating the video image and physiological data;
    said second, consultive station, responsive to at least said first bedside station and including means for receiving and displaying together said combined video image and physiological data from each of said first stations.

21. The patient monitoring system of claim 20 further including an audio system for interactive audio communication between said stations contemporaneously with transmission of the physiological data and video image.

22. A patient monitoring system with integrated physiological and video communication including at least a bedside station, a central station and a consultive station, comprising:
    a local service station including an archival storage and retrieval medium and
    means, responsive to one of said other stations, for retrieving from said archival medium historical patient data and transmitting it to and displaying it at said other station.

23. The patient monitoring system of claim 22 in which said means for retrieving and transmitting includes a fiber optic communication system.

24. An ambulatory patient monitoring system comprising:
    a communication system including a transceiver unit associated with each patient and including means for sensing physiological data from the patient; a plurality of monitoring and location nodes including means for broadcasting through each node an identification of a patient to be monitored; means for interrogating said patient's transceiver circuit through at least one of said nodes for acquiring the sensed physiological data;
    a central station for receiving and displaying the physiological data from said communication system; and

a communication network for interconnecting said central station and said communication system.

25. The patient monitoring system of claim 24 in which said communication system includes means for reactuating said means for broadcasting when said patient is out of range of at least one of said nodes.

26. The patient monitoring system of claim 24 in which said communication network is a fiber optic communication network.

27. The patient monitoring system of claim 24 in which said communication system includes an r.f. communication system.

*Fig. 1*

Fig. 2

EP 0 505 627 A2

Fig. 3

<u>36</u>

TV scan display
integrated patient image curves/
vital sign numerics
also data from Network

74a

70b

52

Fiberoptic
communication
network
interface

82b — Image display control

Waveform display controller

Numeric/ text/ graphic controller

78b

84b — Video decompress

Anti-alias curve draw

86b    80b

μProcessor system

M

P    P

100

60b

Operator keyboard

72b

Audio digitize ¢ compand

MIC

32bbb

Audio decompand ¢recover

32aa

32b

Speaker

*Fig. 4*

400

Archival medium

μProcessor control

60d

402

52d — Network interface

*Fig. 8*

15

Ⓐ      Ⓑ    Ⓒ    Ⓓ

**Shared Memory System**

52

332 →

334

| Physiological and Misc. Message Memory | Audio Message Memory<br>Send ┊ Recv. | Image Message Memory |
|---|---|---|

336

338

*Fig. 5*

Sheet 1 of 3

| FDDI System Interface (FSI)<br>Motorola MC68839 | | Mem Control PAL and Routing Control |
|---|---|---|

342

User Communic. Control Bus (UCC Bus)

340

Adapts FSI to User to UCC Bus

| Media Access Control Circuit (MAC)<br>Motorola MC68838 |
|---|

344

Content Addressable Memory (CAM) Interface

| Elasticity and Link Manage Physical Layer Circuit (ELM)<br>Motorola MC68837 |
|---|

346

356

350

358

360

348    352    354

| Pin diode | Amplifier | FDDI Clock Gen.,Timing and Data Recovery Unit (FCG)<br>Motorola MC68836 | Driver | LED |
|---|---|---|---|---|

from Fiber Optic Cable

Available as module from H.P., Honeywell, Sumimeto, etc.

→ to Fiber Optic Cable

EP 0 505 627 A2

Fig. 5 Sheet 2 of 3

to/from Fig. 5 Sheet 3

to/from Fig. 5 Sheet 1

Voice Audio to Speaker/Mirophone — OUT / IN

Text and Curve Display Mem. (Video RAM) — 78b, 80b

70b

Mem. Cntl.

370

Anti-alias Curve Draw Engine — 366

MC145554 or MCI45557 PCM Codec/Filter — 374

MC145532 ADPCM Transcoder — 378

F — F — O — 368

Audio Timing — 376

Serial/Parallel Conv.

Parallel/Serial Conv.

32aaa,bbb

78b,82b

Graphic Processor TMS34020 — 364

CRT Timing

80b, 86b

Image Control Processor MC68030 — 380

Audio Control Processor MC68030 — 372

Main Control Physiological Data & Misc. Processor MC68030 — 362

from Keyboard

330

Node Processor MC68030 System for Station Management and Control

60b

A   B   C   D   E   F   G

Fig. 5 Sheet 3 of 3

**Fig. 6**

Sheet 1 of 4

34

220 — Audio Control Processor MC68030 System

232 — Audio Timing / Serial/Parallel / Parallel/Serial

MC145532 ADDCM Transcoder

MC145554 or MC145557 PCM Codec/Filter

Voice Audio to Speaker/Amp from Amp/Microphone

32aa,bb — 228 — 230

222 — Main Control Physiological Data & Misc. Processor MC68030 System

Graphic Processor TMS34020 — 78a — 214

216 — Mem. Cntl.

80a, 78a — Text and Curve Display Mem. (Video RAM) — 218

210 — FIFO — 86a

Anti-alias Curve Draw Engine — 212

224 — Image Control Processor MC68030 System

226 — Node Processor for Station Management and Control MC68030 System

60a

206 — 204 — 208 — D — E — 292

to/from Fig. 6 Sht. 2

IMSG180 MUX/Color Lookup Triple DAC — 202

70a — 200 — R  G  B  Video to Monitor

A — B — C

to/from Fig. 6 Sht. 3

19

Fig. 6  Sheet 2 of 4

_Fig. 6_ Sheet 3 of 4

to/from Fig.6 Sht 1

(B) (C) (A)

Physiologic. Data and Misc. Message Memory — 256

Audio Message Memory — 258

290

UCC Bus → to/from Fig.6 Sht.2

Mem.Cntl & Routing — 288

FSI MC68839 — 284

F

to/from Fig.6 Sht 4

Cam Interface

MAC MC68838 — 286

G

ELM MC68837 — 282

ELM

ELM

ELM

FGC MC68836 — 280

FCG

FCG

FCG

270 — Amplif.

Driver — 278

Amplif.

Driver

Amplif.

Driver

Amplif.

Driver

272 — Pin diode

LED — 276

Pin diode

LED

Pin diode

LED

Pin diode

LED

50

268 274

Fiber Cable to Single Attachment Station — 260

262

264

266

EP 0 505 627 A2

*Fig. 6* Sheet 4 of 4

EP 0 505 627 A2

Fig. 7

Sheet 1 of 3

EP 0 505 627 A2

*Fig. 7*

Sheet 2 of 3

to/from Fig. 7 Sheet 1

(A)

142 — Memory Control and Routing Control — Adapts FSI to UCC Bus

Content Addressable Memory (CAM) Interface

146 — FDDI System Interface (FSI) MC68839

148 — Media Access Control Circuit (MAC) MC68838

150 — Elasticity and Link Manage Physical Layer Circuit (ELM) MC68837

152 — FDDI Clock Gen., Timing and Data Recovery Unit (FCG) MC68836

158 — LED

156 — Driver

154

162 — Amplifier

160 — Pin diode

24

Fig. 7
Sheet 3 of 3

Patient name 1    H.R.
                  R.R.
                  Temp

                  _412_

Patient name 2    H.R.  72
                  R.R   2.7
                  Psys  95
                  Pdys  70
                  Temp 105

                  414

                  H.R.
                  Temp

                  H.R.
                  R.R

                  410

416          • 4 Patient          418
             • ECG ¢ Patient parameter overlay

_Fig. 9_

412                         420

H.R.        69 b/m
R.R.        21 br/m
P. syst     169 mm
P. diast    95 mm
Temp        99.2°C

ECG

P

1 HR
TREND

• Single patient 1/4 screen
• Full curves
• Parameters

_Fig. 10_

Fig. 11

## Cellular Node

Network 450

Network interface — 458

452

μProcessor (timer) — 460

Transmitter/ receiver — 462

Signal strength measurement circuit — 464

*Fig.* 12

466

Battery pack

Transmitter/ receiver — 468

472

μProcessor — 470

Memory

456

Sensor, FE — 474

Sensor — 476

## Mobile Patient Data Acquisition

480 — Broadcast to all cellular nodes I.D. of patient to be monitored

482 — Select strongest reply

484 — Switch selected node to maintenance mode

486 — Transfer date

488 — Patient out of range

*Fig.* 13

EP 0 505 627 A2